# EUROPÄISCHE PATENTSCHRIFT

(11) **EP 2 384 712 B1**
(45) Veröffentlichungstag und Bekanntmachung des Hinweises auf die Patenterteilung: **12.03.2014**
(21) Anmeldenummer: 10162271.0
(22) Anmeldetag: 07.05.2010
(51) Int. Cl.: A61B 17/86

(54) **Knochenschraube**
Bone screw
Vis à os

(43) Veröffentlichungstag der Anmeldung: 09.11.2011
(73) Patentinhaber: Pastl, Klaus, 4040 Lichtenberg (AT)
(72) Erfinder: Pastl, Klaus, 4040 Lichtenberg (AT)
(74) Vertreter: KLIMENT & HENHAPEL

(56) Entgegenhaltungen:
- EP-A1- 0 654 248
- EP-A1- 1 369 087
- WO-A1-2004/010889
- WO-A2-2009/120969
- US-A1- 2003 158 556
- US-A1- 2010 023 064

## Beschreibung

Die Erfindung betrifft eine Knochenschraube bestehend aus einem Schraubenkopf und einem, mit einem Außengewinde versehenen Bolzenschaft aus allogenem Knochenmaterial für die chirurgisch operative Osteosynthese, gemäß dem Oberbegriff von Anspruch 1. Sofern sich das Außengewinde eines solchen Bolzens über die gesamte Längserstreckung des Bolzens erstreckt, kann auch von einem Gewindestift gesprochen werden. Ist der Bolzen ferner mit einem gewindelosen Kopf versehen, kann auch von einer Knochenschraube gesprochen werden. Im Folgenden wird vorzugsweise von Knochenschrauben gesprochen, wobei von dieser Bezeichnung auch Ausführungen in Form eines Gewindestifts umfasst sein sollen.

Knochenschrauben für die chirurgisch operative Osteosynthese werden in herkömmlicher Weise aus Metall bzw. Metalllegierungen gefertigt. Ferner sind Knochenschrauben aus resorbierbarem Material, etwa Polyglykolid und Polylaktid, bekannt, sowie Schrauben aus xenogenem Knochen. Schrauben dieser Art wurden etwa in der EP 1 369 087, WO 2009/120969, WO 2004/010889, US 2010/023064, EP 0 654 248, US 2011/160728, US 2010/030271 und der US 2003/158556 offenbart, wobei die EP 1 369 087 als nächstkommender Stand der Technik betrachtet wird.

Knochenschrauben dieser Art weisen in der chirurgischen Praxis allerdings mehrere Nachteile auf. So müssen etwa Schrauben aus Metall bzw. Metalllegierungen einerseits durch eine Zweitoperation wieder entfernt werden, und unterliegen andererseits Veränderungen durch Korrosion. Damit erhöhen sich die Kosten im Gesundheitssystem. Außerdem besteht ein zusätzliches gesundheitliches Risiko für jeden Patienten durch eine neuerliche Operation, das bei Schrauben aus allogenem Knochen nicht besteht. Sämtliche resorbierbaren Materialien im menschlichen oder tierischen Körper wiederum bilden zwar je nach Material eine mehr oder weniger feste Brücke zwischen den zu osteosynthetisierenden Knochen, werden aber aufgelöst, was die Festigkeit der Osteosynthese der betroffenen Knochen negativ beeinflusst. Des Weiteren führen manche resorbierbare Synthesematerialien während ihres Abbaus zu großen Osteolysen in dem umgebenden Knochen, also zu einem Wegweichen des Empfängerknochens von der Schraube. Xenogene (speziesfremde) Materialien wiederum führen zu Abstoßungsreaktionen und sind für die Osteosynthese auch deshalb ungeeignet, weil sie in den umgebenden Empfängerknochen nicht eingebaut, sondern abgestoßen und abgebaut werden, auch wenn das Eiweiß im Knochen vorher durch Hitze denaturiert wurde. Weiters trägt auch der unterschiedliche Elastizitätsmodul von boviner Corticalis und humaner Corticalis (Mensch ca. 16.000 N/mm², Rind ca. 22.000 bis 24.000 N/mm²) dazu bei, dass humanes Material wesentlich besser einheilen kann. Formfestigkeit und Elastizitätsmodul des corticalen Knochens sind dabei speziesabhängig.

Schrauben aus allogenem Knochen (Femur und Tibia-Corticalis) verfügen hingegen über mehrere Vorteile. Sie werden ohne Abstoßungsreaktion vaskularisiert und umgebaut, und sind vor allem für Osteosynthesen dort geeignet, wo kleine Knochenfragmente zusammengefügt werden müssen, da durch die Schraube bereits bei der Operation eine tragende Knochenbrücke entsteht, die sich vom Zeitpunkt der Operation an verbessert, indem sie sich umbaut und voll in den lebenden Knochen integriert und eingebaut wird. Schrauben mit einem Durchmesser von 3-4 mm etwa werden innerhalb von 2 Monaten vollständig mit Gefäßen durchwachsen. Im Gegensatz dazu stellen Metallschrauben eher ein Hindernis für die Knochenneubildung dar, insbesondere verringern sie durch deren bloße Präsenz die zur Verfügung stehende Oberfläche, die für die Knochenheilung vorhanden wäre. Abbaubare Materialien wiederum haben ihre maximale Festigkeit zum Zeitpunkt der Operation. Für sie gelten dieselben Nachteile wie für die Metallschrauben, des Weiteren nimmt die Festigkeit rapide ab, sobald der Abbauprozess eintritt, wodurch die zu osteosynthetisierende Knochenstelle zumindest vorübergehend wieder eine Schwächung erfährt.

Des Weiteren kann bei Knochenschrauben aus allogenem Knochen eine Zweitoperation zur Entfernung des Osteosynthesematerials entfallen, da der Knochen vollständig in eigenen Knochen umgewandelt (nicht resorbiert!) wird. Für den Patienten verringert sich somit das Operationsrisiko, für das Gesundheitssystem verringern sich gezwungenermaßen die Kosten. Schrauben aus allogenem Knochen stören auch nicht bei der Anwendung bildgebender Verfahren, im Gegensatz zu Metallschrauben, die störende Artefakte im MRI und CT hinterlassen. Auch Nachuntersuchungen sind problemlos möglich, und lassen eine bessere Beurteilung des Heilungserfolges zu.

Allerdings ist es für eine Anwendbarkeit von Schrauben aus allogenem Knochen in der chirurgischen Praxis notwendig, deren Gewinde hinsichtlich Eindrehwiderstand und Festigkeit der Schraube zu optimieren. Da sie aus allogener, humaner Corticalis gewonnen werden, ist nicht zu erwarten, dass Gewindeformen, wie sie von Knochenschrauben aus Metall bekannt sind, ohne weiteres übertragbar sind. Tatsächlich besteht hierin auch ein Grund dafür, dass Schrauben aus autologem oder allogenem Knochen bislang in der chirurgischen Praxis noch wenig Verbreitung gefunden haben.

Andererseits ist der Einsatz von Schrauben aus allogenem Knochen nicht gänzlich unbekannt, und wurden bereits für unterschiedliche Anwendungen vorgeschlagen. So wurde etwa versucht, im Rahmen der Rekonstruktion von Kreuzbandrupturen Schrauben aus allogenem Knochen zur anatomischen Fixation der eingesetzten Transplantate zu verwenden. Diese Schrauben werden auch als Interferenzschrauben bezeichnet, da das eingesetzte Transplantat über eine Übermaßpassung der Schraube im spongiösen Knochen befestigt wird. Interferenzschrauben werden daher in Form von Madenschrauben gefertigt, mit vergleichsweise geringer Länge und großem Außendurchmesser, der sich zur Schraubenspitze zunehmend verjüngt. Das Gewinde von Interferenzschrauben ist asymmetrisch ausgeführt, um sie gegenüber Zugkräften belastbarer zu gestalten. Interferenzschrauben dieser Art sind aber für die operative Osteosynthese ungeeignet, da sie sich kaum zum Verbinden zweier getrennter Knochenteile eignen.

Eine weitere Schwierigkeit ergibt sich daraus, dass bekannte Knochenschrauben in vorgegebenen Standardlängen gefertigt werden. Eine maßgefertigte Anpassung an die jeweilige Knochenbruchstelle erfolgt nicht. Dadurch muss der Operateur mitunter auf Knochenschrauben zurückgreifen, die in ihrer Länge nicht optimal auf die jeweilige Bruchstelle abgestimmt sind, und entweder den Knochen nicht vollständig durchsetzen, oder ihn überragen.

Schließlich zeigt sich bei allen bekannten Knochenschrauben der Nachteil, dass sie als Zugschrauben ausgeführt sind, also im Zuge des Einschraubens die beiden zu verbindenden Knochenteile zueinander ziehen. Der Operateur setzt hierzu in eine vorgefertigte Bohrung die Knochenschraube ein, die in der Regel über ein selbstschneidendes Gewinde verfügt, wobei die Knochenschraube nach Durchtritt durch den ersten Knochenteil und Eintritt in den zweiten Knochenteil beide Knochenteile aneinander presst. Dadurch erfolgt eine Druckbelastung auf die betroffenen Knochenteile, die das Knochenmaterial schädigen kann.

Es ist somit das Ziel der Erfindung, für Schrauben aus allogenem, kortikalen Knochen ein optimales Design für den speziellen Einsatzzweck der operativen Osteosynthese zu finden, sodass eine optimale Festigkeit der Knochenverbindung erreicht wird. Dabei soll eine Kompression der zu verbindenden Knochenteile vermieden werden, also Druckbelastungen auf die betroffenen Knochenteile im Zuge des Einsetzens der Knochenschraube. Des Weiteren soll die erfindungsgemäße Knochenschraube an die Anforderungen der jeweiligen Knochenbruchstelle flexibel anpassbar sein.

Diese Ziele werden durch die Merkmale von Anspruch 1 erreicht. Anspruch 1 bezieht sich auf eine Knochenschraube bestehend aus einem Schraubenkopf und einem, mit einem Außengewinde versehenen Bolzenschaft aus allogenem, kortikalen Knochenmaterial für die chirurgisch operative Osteosynthese, bei dem erfindungsgemäß vorgesehen ist, dass der Bolzenschaft nach Abtrennung des bündig mit der Knochenoberfläche abzuschneidenden, schraubenkopfseitigen Abschnittes über seine gesamte Längserstreckung zylindrisch ausgeführt ist, sowie mit einem symmetrischen Spitzgewinde mit konstantem Gewindedurchmesser versehen ist, wobei der Bolzenschaft nach Abtrennung des bündig mit der Knochenoberfläche abzuschneidenden, schraubenkopfseitigen Abschnittes über seine gesamte Längserstreckung zumindest einen Gewindegang pro Millimeter aufweist. Die vergleichsweise hohe Anzahl an Gewindegängen über den gesamten Bolzenschaft hat sich im Rahmen der operativen Osteosynthese für eine gute Festigkeit der Knochenverbindung als entscheidend herausgestellt. Ein Knochen besteht nämlich im Wesentlichen aus der äußeren Kortikalis, die über vergleichsweise höhere Festigkeit verfügt, als die innen liegende, schwammartige Spongiosa. Wird eine Bruchstelle eines Knochens mithilfe einer Knochenschraube fixiert, so quert sie den gesamten Knochenaufbau, also die vergleichsweise dünne Kortikalis, sowie die innen liegende Spongiosa. Es hat sich nun herausgestellt, dass die Festigkeit der Knochenverbindung im Wesentlichen vom Anteil des Bolzenschaftes, der in der Kortikalis zu liegen kommt, abhängt, und der Beitrag der Verschraubung entlang des spongiösen Knochens nur gering ist. Durch die erfindungsgemäß vorgesehene Anzahl an Gewindegängen pro Millimeter wird sicher gestellt, dass die Kortikalis in Verbindung mit einer entsprechend hohen Zahl an Gewindegängen steht, um die Kontaktoberfläche zwischen Knochenschraube und Empfängerknochen zu maximieren, und damit die Festigkeit zu optimieren.

Hierbei ist es aber auch wesentlich, dass der Bolzenschaft über seine gesamte Längserstreckung zylindrisch ausgeführt ist, also insbesondere über keine sich verjüngende Spitze, oder einen konisch ausgeführten, schraubenkopfseitigen Abschnitt verfügt, und über seine gesamte Längserstreckung mit einem konstanten Gewindedurchmesser versehen ist. Auf diese Weise wird sicher gestellt, dass die Knochenschraube in jedem Zehntelmillimeter der vergleichsweise dünnen Kortikalis mit einer entsprechend großen Oberfläche in die Kortikalis eingreift und somit guten Halt findet. Eine sich verjüngende Spitze würde etwa einen deutlich verringerten Halt in der Kortikalis aufweisen, oder sie wird soweit in den Knochen eingeschraubt, dass die Spitze den Knochen überragt. Die erfindungsgemäße Knochenschraube wird soweit eingeschraubt, bis das zylindrische Schraubenende die Knochenoberfläche erreicht, und lediglich der schraubenkopfseitige Abschnitt wird bündig mit der Knochenoberfläche abgeschnitten. Hierzu ist es auch wesentlich, dass der Bolzenschaft der erfindungsgemäßen Knochenschraube *über seine gesamte Längserstreckung* mit einem symmetrischen Spitzgewinde versehen ist, um insbesondere sicherzustellen, dass sich auch ein Gewindeanteil in der schraubenkopfseitigen Kortikalis befindet. Die erfindungsgemäße Knochenschraube vermeidet dadurch, dass sich ein gewindefreier Abschnitt der Knochenschraube in der Kortikalis befindet, wie er mitunter bei herkömmlichen Knochenschrauben vorgesehen ist, nämlich als konisch ausgeführter, schraubenkopfseitige Abschnitt, der im Knochen versenkt wird. Ein solcher gewindefreier Abschnitt weist in der Praxis zwei Nachteile auf. Zum Einen weisen Knochenschrauben dieser Art eine verringerte Rotationsstabilität und geringere Selbsthemmung auf, und zum Anderen wird ein Einwachsen der Knochenschrauben in diesem gewindefreien Abschnitt erschwert, sodass in der Praxis immer wieder Pseudoarthrosen in diesen Bereichen auftreten.

Die erfindungsgemäße Knochenschraube hingegen stellt sicher, dass sich jedenfalls Gewindeabschnitte in der Kortikalis der Knochenbruchstellen befinden. Sie kann in weiterer Folge schraubenkopfseitig überall bündig mit der Knochenoberfläche abgeschnitten werden. Die erfindungsgemäße Knochenschraube muss daher nur mehr in einer einzigen Standardlänge hergestellt werden, da sie ohne weiteres für die jeweilige Anwendung entsprechend abgeschnitten werden kann.

Des Weiteren ist erfindungsgemäß vorgesehen, dass die Knochenschraube mit einem symmetrischen Spitzgewinde versehen ist. Die der Einschraubrichtung zugewandte Flanke eines Gewindeganges weist somit denselben Winkel auf, wie seine abgewandte Flanke. Sie übt daher keine Zugwirkung auf den Knochen aus. Im Zuge der operativen Osteosynthese wird stattdessen die Fixierung und Kompression der zu verbindenden Knochenteile zunächst durch entsprechende chirurgische Instrumente sicher gestellt. In weiterer Folge wird eine Kernbohrung gesetzt, in das ein Gewinde geschnitten wird. Die erfindungsgemäße Knochenschraube kann nun eingebracht werden, wobei die Knochenschraube die Knochenteile in einem stabilen Zustand mit vorgegebener Distanz hält, ohne dabei eine Kompression auszuüben, da ein symmetrisch ausgeführtes Spitzgewinde kaum Zugbelastungen zulässt. Die erfindungsgemäße Knochenschraube stellt daher keine Zugschraube dar. Die Druckbelastung auf das Muttergewinde und auf das Schraubengewinde wird reduziert, bzw. auf Grund des symmetrischen Flankenwinkels zumindest auf beide Gewindeabschnitte gleich verteilt. Somit kann auf diese Art und Weise am besten Drucknekrosen des Knochens vorgebeugt bzw. entgegengewirkt werden. Aufgrund der hohen Gewindezahl und der damit verbundenen Reibungskräfte ist eine hohe Rotationsstabilität der Knochenschraube und vor allem eine große Oberfläche für die Knochenheilung gegeben.

Das Verhältnis der Gewindetiefe zum Gewindedurchmesser kann etwa zwischen 0.10 und 0.15 betragen, und das Produkt aus diesem Verhältnis und der Anzahl der Gewindegänge pro Millimeter zwischen 0.10 und 0.30. Diese Gewindeparameter stellen eine sehr hohe Anzahl an Gewindegängen entlang des Bolzenschaftes sicher, wobei die Gewindetiefe im Vergleich zum gesamten Schraubendurchmesser klein gewählt ist. Der vorgesehene Wertebereich von 0.10 bis 0.30 bewährt sich insbesondere bei Schrauben aus kortikalem Knochenmaterial, die für die operative Osteosynthese besonders geeignet ist. Hinsichtlich der Gewindeform kann überdies vorgesehen sein, dass das Verhältnis der Steigung des Gewindes zum Gewindedurchmesser zwischen 0.05 und 0.25 beträgt.

Die Erfindung wird Im Folgenden anhand eines Ausführungsbeispiels mithilfe der beiliegenden Figuren näher erläutert. Hierbei zeigen die
Fig. 1 einen Gewindeabschnitt eines Spitzgewindes zur Erläuterung der maßgeblichen Gewindeparameter, die
Fig. 2 einen schematischen Querschnitt durch einen Teilbereich einer Knochenschraube mit, im Vergleich zum Bolzenschaft verjüngt ausgeführtem Kopf, die
Fig. 3 eine schematische Darstellung einer erfindungsgemäßen Knochenschraube, und die
Fig. 4 eine schematische Darstellung der Bruchstelle eines Knochens mit einem eingesetzten Bolzen gemäß der Erfindung im Rahmen einer operativen Osteosynthese.

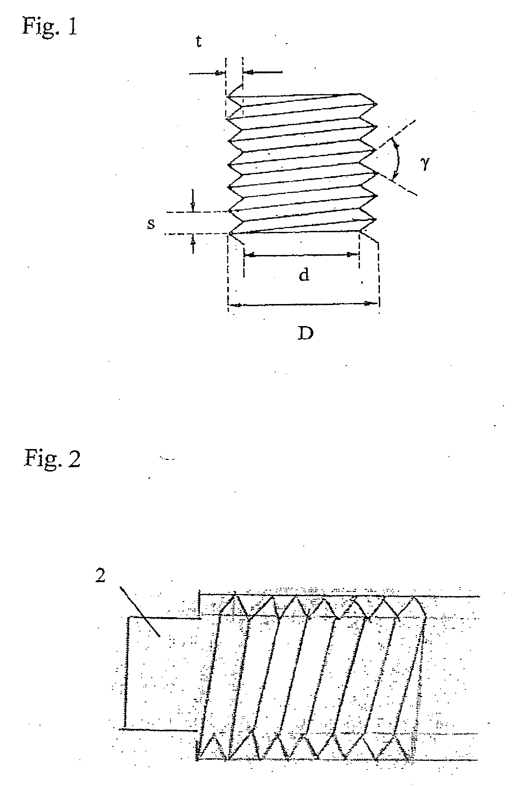

Anhand der Fig. 1 werden zunächst die für die Erfindung maßgeblichen Gewindeparameter erläutert. Es handelt sich hierbei um den Gewindedurchmesser D und den Kerndurchmesser d, wobei durch deren Differenz die Gewindetiefe t festgelegt ist, sowie die Steigung s des Gewindes, wobei die Steigung s jener Weg ist, der durch eine Umdrehung der Schraube zurück gelegt wird, also der Abstand zwischen zwei Gewindespitzen, der bei metrischen Gewinden in Millimeter angegeben wird. Der Kehrwert der Steigung s entspricht in der Regel der Anzahl der Gewindegänge G pro Längeneinheit, also bei metrischen Gewinden der Anzahl der Gewindegänge G pro Millimeter. Ein weiterer Parameter ist der Flankenwinkel γ, der sich wiederum aus der Gewindetiefe t und der Steigung s ergibt.

Je größer der Flankenwinkel γ bei gleich bleibender Gewindetiefe t ist, desto weniger Gewindegänge G pro mm stehen für die Knochenverankerung zur Verfügung, und desto geringer ist damit auch die Kontaktfläche der kommunizierenden Knochen. Dadurch nimmt die Festigkeit, insbesondere auch die Primärstabilität, der Schraube ab. Auch das Bruchdrehmoment der Knochenschraube fließt darin ein, da metallische Schrauben ein wesentlich höheres Bruchdrehmoment aufweisen als Corticalisschrauben. Daraus können hinsichtlich der Festigkeit der Schraube die optimale Steigung s, der optimale Flankenwinkel γ und die optimale Gewindetiefe t abgeleitet werden.

Die vorliegende Erfindung betrifft die weiter gehende Optimierung von Knochenschrauben 1 aus allogenem Knochen hinsichtlich ihrer Eigenschaften im Rahmen der operativen Osteosynthese, insbesondere in Bezug auf die Stabilität der erzielten Knochenverbindung.

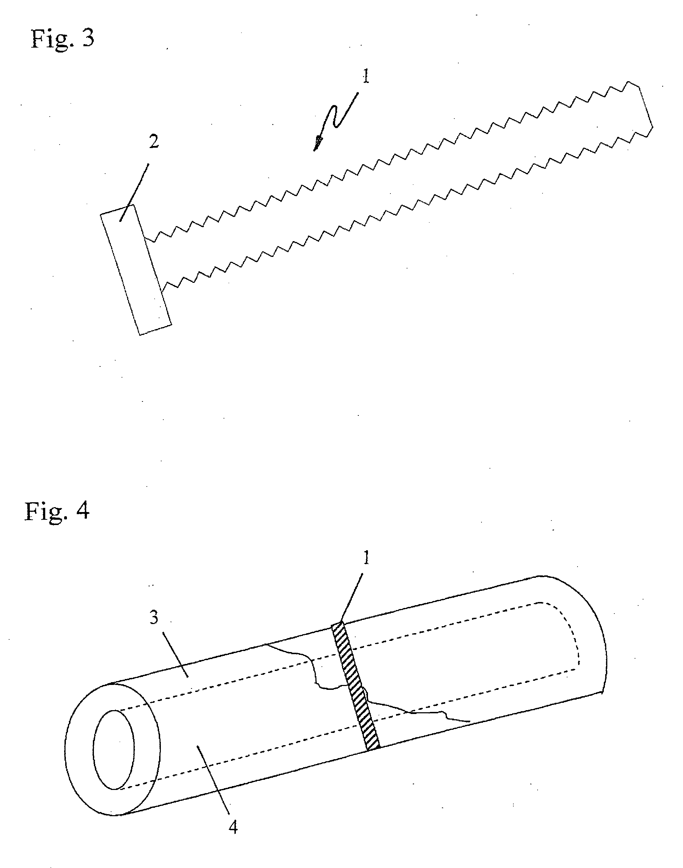

Wie bereits ausgeführt wurde, werden erfindungsgemäß symmetrische Spitzgewinde vorgesehen, um keine Kompression auf die zu verbindenden Knochenteile auszuüben. Da außerdem die Festigkeit der Knochenverbindung im Wesentlichen von der Anzahl der Gewindegänge in der Kortikalis 3 abhängt (siehe Fig. 4), und der Beitrag der Verschraubung entlang des spongiösen Knochens 4 nur gering ist, wird erfindungsgemäß zumindest ein Gewindegang pro Millimeter entlang des gesamten Bolzenschaftes vorgesehen um sicherzustellen, dass die Kortikalis 3 in Verbindung mit einer entsprechend hohen Zahl an Gewindegängen steht. Bei einem Verhältnis von Gewindetiefe t zu Gewindedurchmesser D zwischen 0.10 und 0.15 ist hierzu etwa ein Produkt von dieser Verhältniszahl und der Anzahl der Gewindegänge G pro Millimeter von 0.10 bis 0.30 vorgesehen.

Das symmetrische Spitzgewinde wird erfindungsgemäß über die gesamte Länge des Bolzenschaftes mit konstantem Gewindedurchmesser D geführt, sodass sich eine Knochenschraube 1 wie in der Fig. 3 dargestellt ergibt.

In der Praxis kann nun etwa so vorgegangen werden, dass der Anwender zunächst den erforderlichen Außendurchmesser der je nach Anwendung benötigten Knochenschraube 1 festlegt. Dieser Außendurchmesser entspricht dem Gewindedurchmesser D. In weiterer Folge kann ein erster Wert zwischen 0.10 und 0.15 ausgewählt werden, woraus die Gewindetiefe t berechnet werden kann. Schließlich kann ein zweiter Wert aus dem Wertebereich von 0.10 bis 0.30 ausgewählt werden, der durch den ersten ausgewählten Wert dividiert wird. Dieser Quotient entspricht der Anzahl der Gewindegänge G pro Millimeter. Dadurch ist das Gewinde festgelegt, da sich die Steigung s und der Flankenwinkel γ unmittelbar aus diesen Parametern ergeben. Mithilfe der so ermittelten Gewindeparameter kann aus allogenem, kortikalen Knochenmaterial die benötigte Knochenschraube 1 gefräst werden.

Die Schraube selbst kann einen Vierkant, Sechskant oder Sternkopf als Schraubenkopf 2 aufweisen (siehe Fig. 3).

Im Zuge der operativen Osteosynthese wird zunächst die Fixierung und Kompression der zu verbindenden Knochenteile durch entsprechende chirurgische Instrumente sicher gestellt. In weiterer Folge wird eine Kernbohrung gesetzt, in das ein Gewinde geschnitten wird. Die erfindungsgemäße Knochenschraube 1 kann nun etwa mit einem Steckschraubenschlüssel mit Drehmoment eingebracht werden, wobei die Knochenschraube 1 die Knochenteile entlang des spongiösen Knochens 4 und der Kortikalis 3 quert, und in einem stabilen Zustand mit vorgegebener Distanz hält, ohne dabei eine Kompression auszuüben, da ein symmetrisch ausgeführtes Spitzgewinde kaum Zugbelastungen zulässt (siehe Fig. 4). Die erfindungsgemäße Knochenschraube stellt daher keine Zugschraube dar. Aufgrund der hohen Gewindezahl und der damit verbundenen Reibungskräfte ist aber dennoch hohe Rotationsstabilität der Knochenschraube gegeben.

Die erfindungsgemäßen Knochenschrauben 1 aus allogenem, kortikalen Knochen stellen somit optimale Schrauben für den speziellen Einsatzzweck der operativen Osteosynthese dar, und erreichen eine optimale Festigkeit der Knochenverbindung. Dabei wird eine Kompression der zu verbindenden Knochenteile vermieden, insbesondere Druckbelastungen auf die betroffenen Knochenteile im Zuge des Einsetzens der Knochenschraube 1. Die Kompression auf die frakturierten Knochenfragmente findet vor dem Einbringen der Knochenschraube 1 durch das chirurgische Spezialinstrumentarium statt. Die Knochenschraube 1 selbst hält die dadurch vorgegebene Knochendistanz in einer stabilen Verbindung, ohne selbst eine weitere Kompression auszuführen. Die Druckbelastung auf das Muttergewinde und auf das Schraubengewinde wird reduziert, bzw. auf Grund des symmetrischen Flankenwinkels γ zumindest auf beide Gewindeabschnitte gleich verteilt. Somit kann auf diese Art und Weise am besten Drucknekrosen des Knochens vorgebeugt bzw. entgegengewirkt werden. Des Weiteren ist die erfindungsgemäße Knochenschraube 1 an die Anforderungen der jeweiligen Knochenbruchstelle flexibel anpassbar.

## Patentansprüche

1. Knochenschraube (1) bestehend aus einem Schraubenkopf (2) und einem, mit einem Außengewinde versehenen Bolzenschaft aus allogenem, kortikalen Knochenmaterial für die chirurgisch operative Osteosynthese, die einen bündig mit der Knochenoberfläche abzuschneidenden, schraubenkopfseitigen Abschnitt, aufweist, wobei der Bolzenschaft nach Abtrennung des bündig mit der Knochenoberfläche abzuschneidenden, schraubenkopfseitigen Abschnittes über seine gesamte Längserstreckung zylindrisch ausgeführt ist, sowie mit einem symmetrischen Spitzgewinde mit einem, über die gesamte Längserstreckung konstanten Gewindedurchmesser (D) versehen ist, **dadurch gekennzeichnet, dass** der Bolzenschaft nach Abtrennung des bündig mit derKnochenoberfläche abzuschneidenden, schraubenkopfseitigen Abschnittes über seine gesamte Längserstreckung zumindest einen Gewindegang (G) pro Millimeter aufweist.

2. Knochenschraube mit Außengewinde nach Anspruch 1, **dadurch gekennzeichnet, dass** das Verhältnis der Gewindetiefe (t) zum Gewindedurchmesser (D) zwischen 0.10 und 0.15 beträgt.

3. Knochenschraube mit Außengewinde nach Anspruch 2, **dadurch gekennzeichnet, dass** das Verhältnis der Gewindetiefe (t) zum Gewindedurchmesser (D) multipliziert mit der Anzahl der Gewindegänge (G) pro Millimeter einen Wert zwischen 0.10 und 0.30 ergibt.

4. Knochenschraube mit Außengewinde nach Anspruch 2, **dadurch gekennzeichnet, dass** das Verhältnis der Steigung (s) des Gewindes zum Gewindedurchmesser (D) zwischen 0.05 und 0.25 beträgt.

## Claims

1. Bone screw (1) consisting of a screw head (2) and a bolt shank provided with an external thread and made of allogenic cortical bone material for surgically operative osteosynthesis, which has a section on the screw head side which is to be trimmed flush with the bone surface, wherein the bolt shank after separation of the section on the screw head side which is to be trimmed flush with the bone surface, is cylindrical over its entire longitudinal extension and is provided with a symmetrical V thread having a constant thread diameter (D) over the entire longitudinal extension **characterized in that** after separation of the section on the screw head side which is to be trimmed flush with the bone surface the bolt shank has at least one thread pitch (G) per millimetre over its entire longitudinal extension.

2. Bone screw having an external thread according to claim 1, **characterized in that** the ratio of the thread depth (t) to the thread diameter (D) is between 0.10 and 0.15.

3. Bone screw having an external thread according to claim 2, **characterized in that** the ratio of the thread depth (t) to the thread diameter (D) multiplied by the number of thread pitches (G) per millimetre is between 0.10 and 0.30.

4. Bone screw having an external thread according to claim 2, **characterized in that** the ratio of the slope (s) of the thread to the thread diameter (D) is between 0.05 and 0.25.

## Revendications

1. Vis à os (1) composée d'une tête de vis (2) et d'une tige de boulon munie d'un filetage extérieur en matériau osseux cortical allogène pour les ostéosynthèses chirurgicales, qui présente une partie du côté de la tête de vis à retailler en affleurement avec la surface de l'os, le tige de boulon étant de forme cylindrique sur toute sa longueur après que la partie du côté de la tête de vis à retailler en affleurement avec la surface de l'os a été détachée, et avec un filetage triangulaire symétrique avec un diamètre de filetage (D) constant sur toute la longueur, **caractérisée en ce que** la tige de boulon présente sur toute sa longueur, après que la partie du côté de la tête de vis à retailler en affleurement avec la surface de l'os a été détachée, au moins une spire de filet (G) par millimètre.

2. Vis à os avec filetage extérieur selon la revendication 1, **caractérisée en ce que** le rapport entre la profondeur du filetage (t) et le diamètre du filetage (D) est compris entre 0,10 et 0,15.

3. Vis à os avec filetage extérieur selon la revendication 2, **caractérisée en ce que** le rapport entre la profondeur du filetage (t) et le diamètre du filetage (D) multiplié par le nombre de spires du filet (G) par millimètre donne une valeur comprise entre 0,10 et 0,30.

4. Vis à os avec filetage extérieur selon la revendication 2, **caractérisée en ce que** le rapport entre le pas (s) du filetage et le diamètre du filetage (D) est compris entre 0,05 et 0,25.
